# EUROPÄISCHE PATENTSCHRIFT

(11) **EP 1 197 182 B1**
(45) Veröffentlichungstag und Bekanntmachung des Hinweises auf die Patenterteilung: **29.11.2006**
(21) Anmeldenummer: 01121133.1
(22) Anmeldetag: 04.09.2001
(51) Int. Cl.: A61B 17/17, A61F 2/46

(54) **Modulare Endoprothese mit Justierwerkzeug und Bohrlehre**
Modular endoprosthesis with adjusting device and drill-guide
Endoprothese modulaire avec dispositif de reglage et guide de perçage

(30) Priorität: 09.09.2000 DE 10044702
(43) Veröffentlichungstag der Anmeldung: 17.04.2002
(73) Patentinhaber: Aesculap AG & Co. KG, 78532 Tuttlingen (DE)
(72) Erfinder: Wallstein, Stefan, 78532 Tuttlingen (DE)
(74) Vertreter: Boehme, Ulrich

(56) Entgegenhaltungen:
- EP-A- 0 465 436
- EP-A- 0 634 154
- EP-A- 0 824 013
- EP-A- 1 099 427
- DE-U- 20 012 609
- FR-A- 2 705 558
- FR-A- 2 732 891
- FR-A- 2 799 115

## Beschreibung

Die Erfindung betrifft eine modulare Endoprothese gemäß dem Oberbegriff des Anspruchs 1. Eine derartige Vorrichtung ist aus der EP-A-0 634 154 bekannt.

Weiterhin betrifft die Erfindung ein Zielgerät zum Auffinden von Querbohrungen im Befestigungsschaft einer derartigen modularen Endoprothese.

Bei Implantatsystemen, die aus einzelnen Modulen aufgebaut sind, ist es möglich, die Formgebung der Prothese optimal an die anatomischen Gegebenheiten anzupassen. Diese Anpassung kann auch intraoperativ erfolgen, indem einfach die passenden Komponenten ausgewählt und dann zusammengefügt werden. Bei bekannten modularen Endoprothesen dieser Art erfolgt die Fügung durch das Einschieben eines Verbindungsschaftes in einen durchgehenden Kanal der jeweils anderen Komponente, die Verbindung kann beispielsweise durch eine selbsthemmende Konusverbindung erfolgen.

Es sind Hüftschaftimplantate zur zementfreien Implantation bekannt, bei denen eine distale Komponente einen in die Markraumhöhle einschiebbaren Befestigungsschaft aufweist und einen Verbindungsschaft, der in eine durchgehende Längsbohrung einer proximalen Komponente eingreift. Wenn diese Komponenten in die Markraumhöhle eingesetzt werden, werden sie gegeneinander verdreht, und es ist häufig schwierig, die Übertragung dieser für den umgebenden Knochen oder den Patienten schädlichen Drehbewegung zu verhindern, außerdem ist es häufig schwierig, die genaue Orientierung der Komponenten um die Längsachse der Endoprothese zu erkennen und einzuhalten.

Diese Orientierung kann zwar bei der proximalen Komponente dadurch kontrolliert werden, daß asymmetrisch seitlich ein Steckzapfen für eine Kugelgelenkkomponente absteht, auf welches ein Gegenhalteinstrument aufgesteckt werden kann (Firmenprospekt Peter Brehm "Die Präzision in Titan für den Menschen"), eine solche Kontrolle ist jedoch bei der Distalkomponente nicht möglich, die vollständig rotationssymmetrisch ausgebildet sein kann und die im Inneren des Markraumes angeordnet wird.

In der FR-A-2,705,558 ist eine Endoprothese beschrieben, deren Schaft entweder in eine Probehülse oder in eine Prothesenhülse eingeschoben werden kann, wobei in beiden Fällen diese Hülsen mit dem Schaft der Prothese drehfest verbunden sind. In keiner Position sind die Hülse und der Schaft frei gegeneinander verdrehbar.

Bei einer weiteren, aus der EP-A-0 824 013 bekannten Endoprothese greift ein Schaft mit einer Verzahnung in der axialen Endposition in einen korrespondierenden Zahnbereich der anderen Komponente ein, so daß eine Verdrehung verhindert wird.

Es ist Aufgabe der Erfindung, bei einer gattungsgemäßen Prothese eine Möglichkeit zu schaffen, die Winkelstellung der Komponenten einer modularen Endoprothese zu kontrollieren.

Diese Aufgabe wird bei einer modularen Endoprothese der eingangs beschriebenen Art erfindungsgemäß dadurch gelöst, daß der Verbindungsschaft an seinem freien Ende einen Außenmehrkant zum drehfesten Ansetzen eines in den Kanal eingeführten Werkzeuges trägt, und daß dieser Außenmehrkant im Inneren des Kanals angeordnet ist, wenn sich die beiden Komponenten in der axialen Endposition befinden.

Es ist möglich, die Komponenten auf diese Weise gegeneinander zu verdrehen, ohne Drehmomente auf den Knochen und den Patienten zu übertragen. Dies ist möglich, solange die beiden Komponenten noch nicht klemmend zusammengefügt sind, also noch nicht fest in axialer Richtung gegeneinander gedrückt sind. Trotzdem nehmen die beiden Komponenten in zusammengesteckter, aber noch nicht fest zusammengeklemmter Form im wesentlichen bereits die relative Endposition in axialer Richtung zu einander ein, zum Zusammenfügen und damit zum Zusammenklemmen genügt eine ganz geringe axiale Verschiebung, die die Relativposition in axialer Richtung praktisch nicht verändert. Vor dem endgültigen Zusammenklemmen kann somit der Operateur die relative Winkelstellung der beiden Komponenten in der gewünschten Weise einstellen, wobei die beiden Komponenten bereits im wesentlichen die endgültige relative Axialposition einnehmen.

Der Kanal der ersten Komponente ist an seinem der zweiten Komponente gegenüberliegenden Ende vorzugsweise durch einen einsetzbaren Stopfen verschließbar.

Bei einer weiteren bevorzugten Ausführungsform ist vorgesehen, daß der Außenmehrkant eine definierte Winkelorientierung relativ zu mindestens einer Querbohrung im Befestigungsschaft der zweiten Komponente aufweist. Diese Querbohrung oder diese Querbohrungen dienen der Aufnahme von Knochenschrauben, die den Röhrenknochen und die Querbohrungen des Befestigungsschafts durchsetzen und diesen dadurch im Röhrenknochen festlegen.

Auf diese Weise ist es möglich, die genaue Orientierung der Querbohrungen über ein angesetztes Zielgerät festzustellen, dessen Orientierung relativ zur zweiten Komponente über die drehfeste Verbindung zwischen Zielgerät und zweiter Komponente festgelegt ist.

Die Erfindung betrifft dementsprechend auch ein Zielgerät zum Auffinden der Querbohrungen im Befestigungsschaft einer Endoprothese, welches dadurch gekennzeichnet ist, daß es ein in den Kanal einführbares, an dem Außenmehrkant drehfest ansetzbares Halteelement aufweist sowie ein mit diesem über ein außerhalb des Kanals verlaufendes Verbindungselement verbundenes, im wesentlichen neben den Komponenten parallel zu dem Kanal verlaufendes Zielelement, welches bei an dem Außenmehrkant angesetztem Halteelement mit den Querbohrungen im Befestigungsschaft ausgerichtete Zielbohrungen aufweist.

Die nachfolgende Beschreibung bevorzugter Ausführungsformen der Erfindung dient im Zusammenhang mit der Erfindung der näheren Erläuterung. Es zeigen:
- Figur 1:: eine Längsschnittansicht durch einen Röhrenknochen mit einer eingesetzten modularen Endoprothese mit angesetztem Zielgerät und
- Figur 2:: eine Ansicht ähnlich Figur 1 im oberen Teil der Endoprothese mit angesetztem Drehwerkzeug.

Die Erfindung wird am Beispiel einer modular aufgebauten Hüftgelenkendoprothese 1 erläutert, es versteht sich aber, daß die Erfindung auch bei anderen modular aufgebauten Prothesen Verwendung finden kann, bei denen zwei Komponenten der Prothese über einen in einen Kanal eingreifenden Verbindungsschaft miteinander zusammengefügt werden.

Die in dieser Zeichnung dargestellte Prothese 1 umfaßt eine erste, proximale Komponente 2, die sich in proximaler Richtung erweitert und seitlich einen Aufsteckzapfen 3 für eine in der Zeichnung nicht dargestellte Gelenkkugel aufweist. Die proximale Komponente 2 wird von einem Kanal 4 durchsetzt, der sich an seinem distalen Ende konisch erweitert und damit eine konische Steckfläche 5 ausbildet.

Die zweite, distale Komponente 6 der Prothese 1 umfaßt im wesentlichen einen länglichen Befestigungsschaft 7 sowie einen sich daran an der proximalen Seite anschließenden Verbindungsschaft 8, der sich an seinem distalen Ende zum proximalen Ende hin konisch verengt und somit eine zur Steckfläche 5 des Kanals 4 komplementäre konische Haltefläche 9 ausbildet, und mit einem sich daran in proximaler Richtung anschließenden zylindrischen Abschnitt 10, an dessen freiem, proximalem Ende bei dem dargestellten Ausführungsbeispiel ein Außenmehrkant 11 in Form eines Zweiflaches angeordnet ist.

Der Befestigungsschaft 7 ist dabei in an sich bekannter Weise so ausgebildet, daß die distale Komponente optimal im Markraum 13 eines Röhrenknochens 14 aufgenommen werden kann, außerdem sind im Befestigungsschaft 7 zwei quer zur Längsachse desselben diesen durchsetzende Querbohrungen 15, 16 angeordnet, die sich im distalen Endabschnitt des Befestigungsschaftes 7 befinden.

Zum Zusammenfügen der proximalen Komponente 2 und der distalen Komponente 6 wird die letztere mit dem Verbindungsschaft 8 in den Kanal 4 eingeschoben, bis die Haltefläche 9 an der Steckfläche 5 eng anliegt. Die Konizität ist dabei so gering, daß beim kräftigen Einschieben Selbsthemmung eintritt.

Wenn die beiden Komponenten 2 und 6 in dieser Weise zusammengefügt sind, befindet sich der Außenmehrkant 11 im Inneren des Kanals 4, ragt also nicht proximal aus der Komponente 2 hervor.

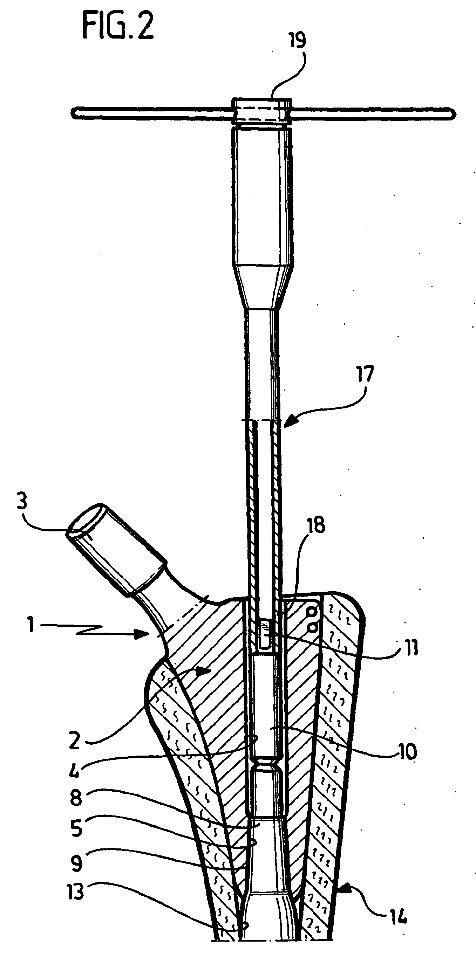

Auf den Außenmehrkant 11 ist ein Steckschlüssel 17 aufsteckbar, der an seinem einen Ende eine Aufsteckhülse 18 trägt, die beim Aufstekken auf den Außenmehrkant 11 eine drehfeste Verbindung herstellt, so daß der Steckschlüssel 17 in der Verlängerung der distalen Komponente 6 drehfest mit dieser verbunden ist, durch die Stellung des Steckschlüssels 17 kann somit auch die Winkelstellung der distalen Komponente kontrolliert werden (Figur 2). Es ist dadurch möglich, die distale Komponente um ihre Längsachse zu drehen oder aber in einer einmal erreichten Position zu fixieren, außerdem kann der Steckschlüssel 17 auch eine Schlagfläche 19 aufweisen, so daß über diese Schlagfläche 19 die distale Komponente in den Markraum 13 eingeschlagen werden kann.

Sowohl beim Fügen als auch beim Lösen der zusammengesteckten Komponenten 2 und 6 kann dieser Steckschlüssel 17 nützlich sein, er wirkt dann als Gegenhalter für die distale Komponente 6, so daß die proximale Komponente 2 durch geeignete Werkzeuge relativ zur distalen Komponente 6 verdreht und in axialer Richtung mit einer Kraft versehen werden kann. Mit dem Steckschlüssel kann bei all diesen Vorgängen die distale Komponente 6 im Röhrenknochen 14 gehalten werden, so daß eine Beschädigung des Röhrenknochens bei der Fügung oder Lösung der Komponenten vermieden werden kann.

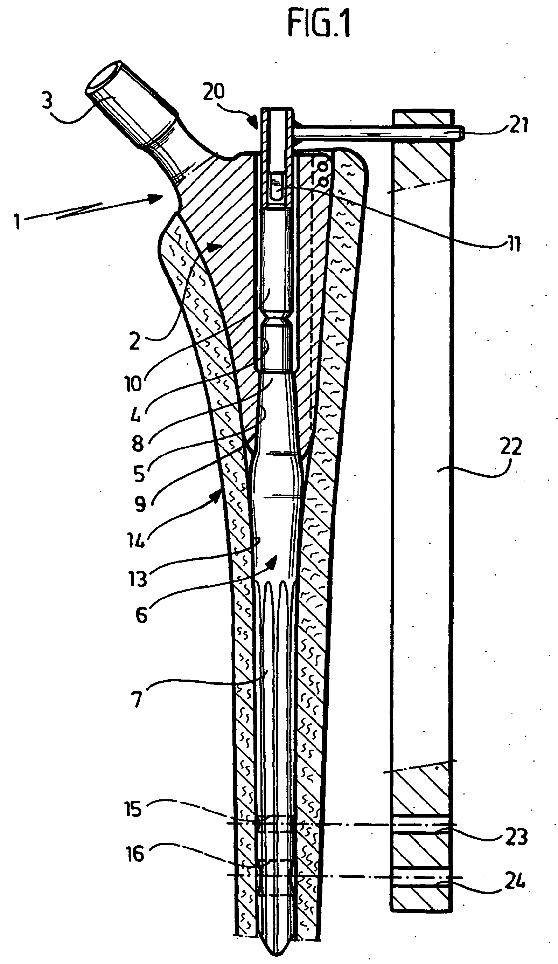

Auf den Außenmehrkant 11 ist ein hülsenförmiges Halteelement 20 aufsteckbar, welches einen Innenmehrkant aufweist und somit drehfest mit dem Außenmehrkant 11 zu verbinden ist (Figur 1). Im aufgesteckten Zustand steht das Halteelement 20 in proximaler Richtung aus dem Kanal 4 hervor und ist dort mit einem radial abstehenden Arm 21 verbunden, an dem seinerseits ein etwa parallel zum Kanal 4 verlaufender, neben dem Röhrenknochen 14 angeordneter Zielbalken 22 festgelegt ist. Dieser erstreckt sich bis zum distalen Ende der distalen Komponente 6 und weist dort zwei Querbohrungen 23, 24 auf, die mit den Querbohrungen 15 und 16 in der distalen Komponente 6 ausgerichtet sind, wenn das Halteelement 20 in einer möglichen Orientierung auf den Außenmehrkant 11 aufgesteckt ist. Es ist dadurch möglich, durch die Querbohrungen 23 und 24 hindurch einen Bohrer so zu führen, daß er beim Durchbohren des Röhrenknochens 14 genau die Querbohrungen 15 und 16 trifft. Durch diese Querbohrungen können dann Knochenschrauben zur Festlegung der distalen Komponente hindurchgeführt werden.

## Patentansprüche

1. Modulare Endoprothese (1) mit mindestens zwei zusammenfügbaren Komponenten (2, 6), wobei die erste Komponente (2) einen durchgehenden Kanal (4) und die zweite Komponente (6) einen in diesen Kanal (4) eingesetzten Verbindungsschaft (8) aufweisen, die beiden Komponenten (2, 6) in einer axialen Endposition durch Verklemmung relativ zueinander fixierbar sind und die zwei Komponenten (2, 6) in der axialen Endposition vor der Fixierung durch Verklemmung relativ zueinander verdrehbar sind, **dadurch gekennzeichnet, daß** der Verbindungsschaft (8) an seinem freien Ende einen Außenmehrkant (11) zum drehfesten Ansetzen eines in den Kanal (4) eingeführten Werkzeuges (17; 20, 21, 22) trägt, und daß dieser Außenmehrkant (11) im Inneren des Kanals (4) angeordnet ist, wenn sich die beiden Komponenten (2, 6) in der axialen Endposition befinden.

2. Modulare Endoprothese nach Anspruch 1, **dadurch gekennzeichnet, daß** der Kanal (4) der ersten Komponente (2) an seinem der zweiten Komponente (6) gegenüberliegenden Ende durch einen einsetzbaren Stopfen verschließbar ist.

3. Modulare Endoprothese nach einem der voranstehenden Ansprüche, **dadurch gekennzeichnet, daß** der Außenmehrkant (11) eine definierte Winkelorientierung relativ zu mindestens einer Querbohrung (15, 16) im Befestigungsschaft (7) der zweiten Komponente (6) aufweist.

4. Modulare Endoprothese (1) nach Anspruch 3, und Zielgerät zum Auffinden der Querbohrungen (15, 16) im Befestigungsschaft (7) der Endoprothese (1), **dadurch gekennzeichnet, daß** es ein in den Kanal (4) einführbares, an dem
Außenmehrkant (11) drehfest ansetzbares Halteelement (20) aufweist sowie ein mit diesem über ein außerhalb des Kanals (4) verlaufendes Verbindungselement (21) verbundenes, im wesentlichen neben den Komponenten (2, 6) parallel zu dem Kanal (4) verlaufendes Zielelement (22), welches bei an den Außenmehrkant (11) angesetztem Halte-element (20) mit den Querbohrungen (15, 16) im Befestigungsschaft (7) ausgerichtete Zielbohrungen (23, 24) aufweist.

## Claims

1. A modular endoprosthesis (1) comprising at least two components (2, 6) which can be joined together, wherein the first component (2) has a continuous channel (4), and the second component (6) has a connecting stem (8) inserted into this channel (4), the two components (2, 6) are fixable relative to one another in an axial end position by clamping, and the two components (2, 6) are rotatable relative to one another in the axial end position before being fixed by clamping, **characterised in that** the connecting stem (8) carries, at its free end, an external polygon (11) for the rotationally secure application of a tool (17; 20, 21, 22) introduced into the channel (4), and **in that** this external polygon (11) is arranged inside the channel (4) when the two components (2, 6) are located in the axial end position.

2. A modular endoprosthesis according to claim 1, **characterised in that** the channel (4) in the first component (2) is closable at its end opposite the second component (6) by an insertable stopper.

3. A modular endoprosthesis according to either one of the preceding claims, **characterised in that** the external polygon (11) has defined angular orientation relative to at least one transverse bore (15, 16) in the fastening stem (7) of the second component (6).

4. A modular endoprosthesis (1) according to claim 3, and a sighting device for locating the transverse bores (15, 16) in the fastening stem (7) of the endoprosthesis (1), **characterised in that** it has a holding member (20) which is introducible into the channel (4) and can be applied to the external polygon (11) in a rotationally secure manner, and a sighting member (22) which is connected to the holding member (20) by a connecting part (21) extending outside the channel (4) and which extends substantially parallel to the channel (4) alongside the components (2, 6) and which, when the holding member (20) is applied to the external polygon (11), has sighting bores (23, 24) which are aligned with the transverse bores (15, 16) in the fastening stem (7).

## Revendications

1. Endoprothèse modulaire (1), comprenant au moins deux composants à assembler (2, 6), dans laquelle le premier composant (2) présente un canal traversant (4) et le deuxième composant (6) présente une tige de liaison (8) placée dans ce canal (4), les deux composants (2, 6) pouvant être fixés l'un par rapport à l'autre par blocage dans une position d'extrémité axiale, et les deux composants (2, 6) pouvant être tournés l'un par rapport à l'autre dans la position d'extrémité axiale avant leur fixation par blocage, **caractérisée en ce que** la tige de liaison (8) porte à son extrémité libre un polygone extérieur (11) pour une application verrouillée en rotation d'un outil (17 ; 20, 21, 22) introduit dans le canal (4), et **en ce que** ce polygone extérieur (11) est disposé à l'intérieur du canal (4) lorsque les deux composants (2, 6) se trouvent dans la position d'extrémité axiale.

2. Endoprothèse modulaire selon la revendication 1, **caractérisée en ce que** le canal (4) du premier composant (2) est refermable par un bouchon à insérer au niveau de son extrémité opposée au deuxième composant (6).

3. Endoprothèse modulaire selon l'une quelconque des revendications précédentes, **caractérisée en ce que** le polygone extérieur (11) présente une orientation angulaire définie par rapport à au moins un alésage transversal (15, 16) dans la tige de fixation (7) du deuxième composant (6).

4. Endoprothèse modulaire (1) selon la revendication 3, et viseur pour retrouver les alésages transversaux (15, 16) dans la tige de fixation (7) de l'endoprothèse (1), **caractérisé en ce qu'**il présente un élément de maintien (20) pouvant être introduit dans le canal (4), applicable de façon verrouillée en rotation au polygone extérieur (11), ainsi qu'un élément de visée (22) relié à celui-ci par l'intermédiaire d'un élément de liaison (21) s'étendant à l'extérieur du canal (4), s'étendant substantiellement à côté des composants (2, 6) en parallèle au canal (4), l'élément de visée présentant des alésages cible (23, 24) alignés sur les alésages transversaux (15, 16) dans la tige de fixation (7) lorsque l'élément de maintien (20) est appliqué au polygone extérieur (11).
